# DEMANDE DE BREVET EUROPEEN

(11) **EP 1 647 263 A1**
(43) Date de publication de la demande: **19.04.2006**
(21) Numéro de dépôt: 05292143.4
(22) Date de dépôt: 13.10.2005
(51) Int. Cl.: A61K 8/40, A61Q 3/00, A61Q 5/06, A61K 8/04, A61K 8/58

(54) **Dispositif aérosol à deux compartiments comprenant des monomères électrophiles et utilisation pour le traitement cosmétique des cheveux**

(30) Priorité: 13.10.2004 FR 0410809
(71) Demandeur: L'OREAL, 75008 Paris (FR)
(72) Inventeur: Rollat-Corvol, Isabelle, 75017 Paris (FR)
(74) Mandataire: Casalonga, Axel

(57) **Abrégé**

La présente invention concerne un dispositif aérosol à deux compartiments comprenant :
dans un premier compartiment, une composition cosmétique comprenant dans un milieu cosmétiquement acceptable, au moins un monomère électrophile, et
dans un deuxième compartiment, un gaz comprimé,
lesdits premier et deuxième compartiments assurant, lors de l'emploi du dispositif aérosol, une séparation mécanique permanente à l'intérieur de celui-ci entre la composition cosmétique et le gaz comprimé.

## Description

La présente invention est relative à un dispositif aérosol à deux compartiments comprenant au moins un monomère électrophile et un gaz comprimé, et à une utilisation de la composition contenue dans le dispositif aérosol pour le traitement cosmétique des cheveux, en particulier pour leur coiffage.

Les compositions de coiffage, telles que les laques et les sprays, conditionnées sous forme de spray aérosol, sont généralement composées d'une phase liquide comprenant, dans un milieu cosmétiquement acceptable aqueux, alcoolique ou hydroalcoolique, au moins un matériau fixant, et d'un agent propulseur qui est un gaz liquéfié sous pression réduite ou dissous dans la phase liquide.

D'autres dispositifs aérosols comprenant une telle phase liquide existent, mais ils comprennent des gaz comprimés pour assurer la propulsion de la phase liquide. Or ces dispositifs à gaz comprimés présentent l'inconvénient d'une perte de pression des gaz au cours du temps. En effet, on observe une fuite des gaz comprimés au cours du temps ou lors d'une mauvaise utilisation du récipient, c'est-à-dire lorsqu'il se retrouve la tête en bas.

Cet inconvénient entraîne une propulsion de moins en moins bonne de la phase liquide au cours du temps et par conséquent une répartition de moins en moins bonne de la phase liquide sur les cheveux.

Un autre facteur pouvant conduire à une mauvaise répartition de la phase liquide à partir de ces dispositifs est la qualité du spray qui se dégrade lorsque l'on cherche à obtenir une fixation la plus importante possible, c'est-à-dire lorsque l'on cherche à augmenter la quantité de polymère que l'on peut dissoudre dans ladite phase liquide.

Dans le domaine du coiffage, les matériaux fixants sont généralement des polymères fixants, c'est-à-dire des polymères filmogènes solubles ou dispersibles dans l'eau et dans l'alcool, tels que les copolymères d'acétate de vinyle/acide crotonique, les résines acryliques anioniques ou amphotères et les polyuréthanes. Ces polymères mélangés à un solvant approprié constituent la phase liquide dans un récipient aérosol, ce qui permet leur pulvérisation sur les cheveux sous forme d'un nuage de gouttelettes dispersées.

Une fois appliquée sur les cheveux, la phase liquide sèche, permettant la formation de soudures nécessaires à la fixation de la coiffure par les matériaux fixants. Les soudures doivent être suffisamment résistantes pour assurer un maintien de la coiffure.

Cependant, elles doivent également être suffisamment fragiles pour que l'utilisateur puisse, en peignant ou en brossant la chevelure, les détruire sans endommager le cuir chevelu ni les cheveux.

La demanderesse a découvert de manière surprenante que la séparation par voie mécanique de la phase liquide du gaz comprimé assurant la propulsion, et l'utilisation de monomères électrophiles tels que décrits dans la demande FR 2 840 208 permettaient de résoudre les problèmes de fuite et de répartition de la phase liquide sur les cheveux, et d'améliorer la fixation et le maintien de la coiffure, c'est-à-dire d'obtenir des soudures de meilleure qualité par rapport à celles obtenues avec un matériau fixant classique tout en conservant de bonnes propriétés cosmétiques telles que douceur et démêlage.

L'invention a donc pour objet un dispositif aérosol à deux compartiments comprenant une composition cosmétique qui comprend au moins un monomère électrophile tel que décrit ci-dessous, dans un premier compartiment, et un gaz comprimé dans un deuxième compartiment, lesdits premier et deuxième compartiments assurant, lors de l'emploi du dispositif, une séparation mécanique permanente à l'intérieur de celui-ci entre la composition cosmétique et le gaz comprimé.

Un autre objet de la présente invention consiste en une utilisation de la composition contenue dans le dispositif pour le traitement cosmétique des cheveux, et plus particulièrement pour le coiffage des cheveux.

L'invention a encore pour objet un procédé de traitement des cheveux comprenant la pulvérisation d'une composition telle que définie ci-dessous sur les cheveux, en présence d'un agent nucléophile tel que défini ci-dessous.

La pulvérisation sur les cheveux d'une composition cosmétique qui comprend au moins un monomère électrophile, au moyen d'un tel dispositif, conduit à une répartition homogène du produit sur les cheveux et à de bons effets cosmétiques.

D'autres objets, caractéristiques, aspects et avantages de l'invention apparaîtront encore plus clairement à la lecture de la description et de l'exemple qui suit.

Le dispositif aérosol à deux compartiments selon l'invention comprend :
- dans un premier compartiment, une composition cosmétique comprenant, dans un milieu cosmétiquement acceptable, au moins un monomère électrophile, et
- dans un deuxième compartiment, un gaz comprimé,
lesdits premier et deuxième compartiments assurant, lors de l'emploi du dispositif, une séparation mécanique permanente à l'intérieur de celui-ci entre la composition cosmétique et le gaz comprimé.

A titre d'exemple de gaz comprimé, on peut notamment citer l'air, l'azote, le gaz carbonique et leurs mélanges, l'air étant particulièrement préféré.

Ledit gaz comprimé est utilisé de préférence sous une pression comprise entre 0,1 et 1,2 MPa, mieux encore entre 0,9 et 1,1 MPa.

Par monomère électrophile, on entend un monomère capable de polymériser par polymérisation anionique en présence d'un agent nucléophile tel que par exemple, les ions hydroxyles (OH⁻) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
(i) les dérivés benzylidene malononitrile (A), le 2-(4-chloro-benzylidene)-malononitrile (A1), le 2-cyano-3-phényl acrylate d'éthyle (B), le 2-cyano-3-(4-chloro-phényl) acrylate d'éthyle (B1) tels que décrits dans Sayyah, J. Polymer Research, 2000, p97 : 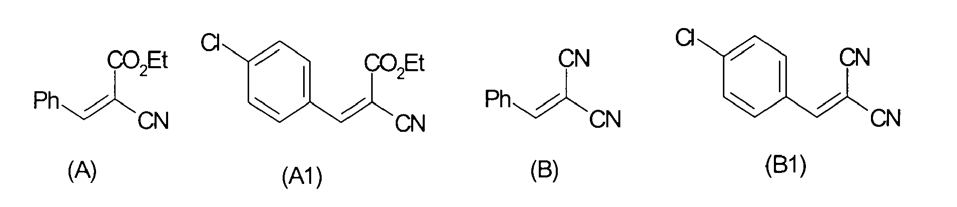
(ii) les dérivés de méthylidenemalonates comme :
   - le 2-méthylene-malonate de diéthyle (C) tel que décrit par Hopff, Makromoleculare Chemie, 1961, p95, par De Keyser, J. Pharm. Sci, 1991, p67 et par Klemarczyk, Polymer, 1998, p173 : 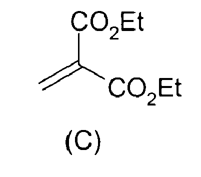
   - le 2-éthoxycarbonylméthyleneoxycarbonyl acrylate d'éthyle (D) tel que décrit par Breton, Biomaterials, 1998, p271 et Couvreur, Pharmaceutical Research, 1994, p1270 : 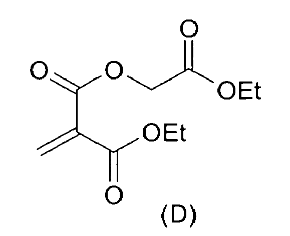
(iii) les dérivés itaconate et itaconimide comme :
   - l'itaconate de diméthyle (E) tel que décrit par Bachrach, European Polymer Journal, 1976, p563 : 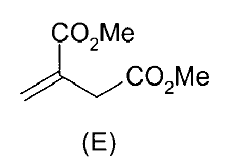
   - le N-butyl itaconimide (F), le N-(4-tolyl) itaconimide (G), le N-(2-ethylphenyl) itaconimide (H), le N-(2,6-diethylphenyl) itaconimide (I) tel que décrits par Wanatabe, J.Polymer Science : Part A :Polymer chemistry, 1994, p2073 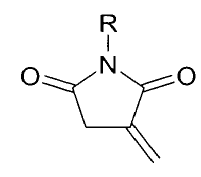 R= Bu (F), 4-tolyl (G), 2-ehylphenyl (H), 2,6-diethyphenyl (I)
(iv) les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O) tel que décrits par Gipstein, J.Org.Chem, 1980, p1486 et les dérivés 1,1-bis-(methylsulfonyl)ethylene (P), 1-acetyl-1-methyl sulfonyl ethylene (Q) , α-(methylsulfonyl) vinyl sulfonate de methyle (R) , α-methylsulfonylacrylonitrile (S) tel que décrits par Shearer, US patent US2748050 : 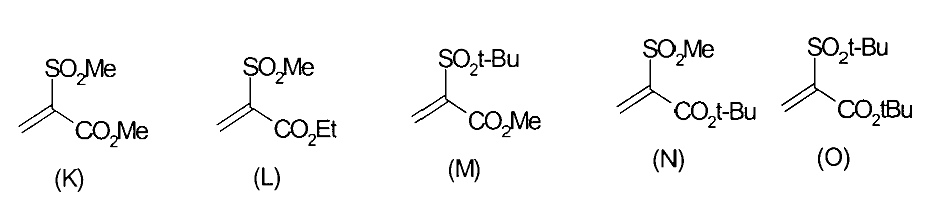 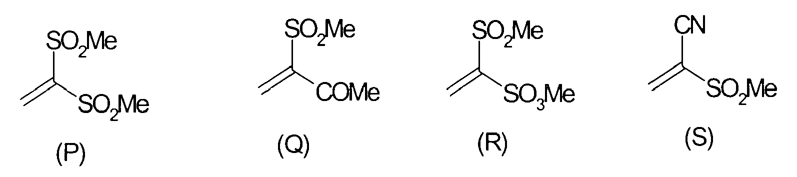
(v) les dérivés méthyl vinyl sulfone (T) et phényl vinyl sulfone (U) tel que décrits par Boor , J.Polymer Science, 1971, p249 : 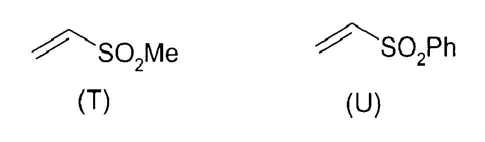
(vi) le dérivé phényl vinyl sulfoxide (V) tel que décrit par Kanga, Polymer preprints (ACS, Divison of Polymer Chemistry), 1987, p322 : 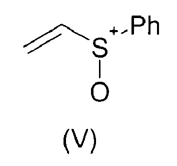
(vii) le dérivé 3-methyl-N-(phenylsulfonyl)-1-aza-1,3-butadiene (W) tel que décrit par Bonner, Polymer Bulletin, 1992, p517 : 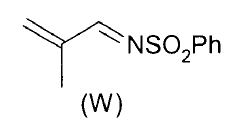
(viii) les dérivés acrylates et acrylamides comme :
   - le N-propyl-N-(3-triisopropoxysilylpropyl)acrylamide (X) et N-propyl-N-(3-triethoxysilylpropyl)acrylamide (Y) tel que décrit par Kobayashi, Journal of Polymer Science, Part A: Polymer Chemistry, 2005, p2754 : 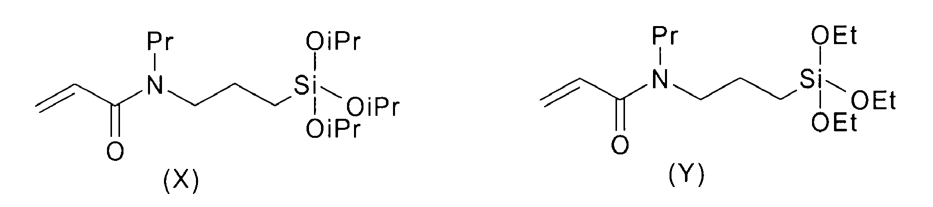
   - le 2-hydroxyethyl acrylate (Z) et le 2-hydroxyethyl méthacrylate (AA) tel que décrits par Rozenberg, International Journal of Plastics Technology, 2003, p17 : 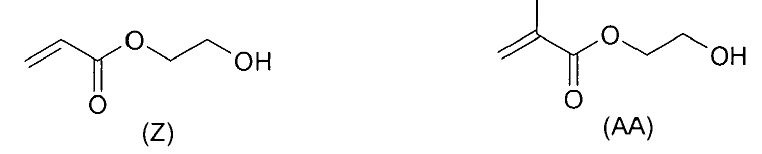
   - le N-butyl acrylate (AB) tel que décrit par Schmitt, Macromolecules, 2001, p2115, et le tert-butyl acrylate (AC) tel que décrit par Ishizone, Macromolecules, 1999, p955 :

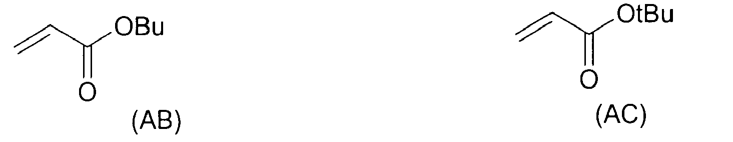

Le monomère électrophile, ou électro-attracteur, utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères électrophiles présentant au moins deux groupes électro-attracteurs, on peut citer les monomères de formule (I) : 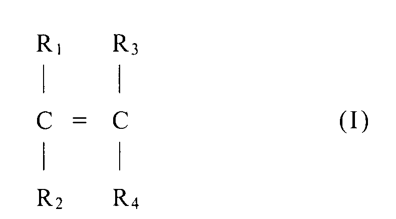 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR,
   - OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, - OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène et un résidu de polymère pouvant être obtenu par polymérisation radicalaire ou par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle, les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou (CH₂)ₙ-(CF₂)ₘ-CHF₂ avec n=1 à 20 et m= 1 à 20.

Les substituants R₁ à R₄ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (II) : 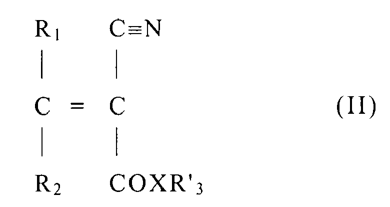 X désignant NH,S,O,
R₁ et R₂ ayant les mêmes significations que précédemment,
R'₃ pouvant désigner un atome d'hydrogène ou un groupe R tel que défini pour la formule (I).

De préférence, X désigne O.

A titre de composés de formule (II), on peut citer les monomères :
a) appartenant à la famille des 2-cyanoacrylates de polyfluoroalkyle en C₁₋₂₀ tels que :
   l'ester 2,2,3,3-tétrafluoropropylique de l'acide 2-cyano-2-propénoïque de formule : 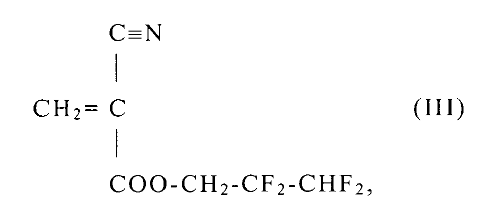
   ou encore l'ester 2,2,2-trifluoroéthylique de l'acide 2-cyano-2-propénoïque de formule : 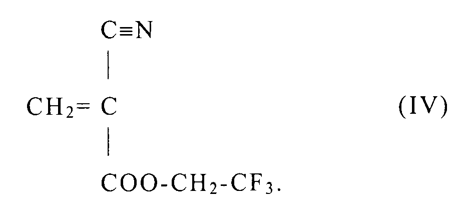
b) les cyanoacrylates d'alkyle en C₁-C₁₀ ou d'(alcoxy en C₁₋C₄)(alkyle en C₁-C₁₀).
   On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères le plus particulièrement préférés sont ceux de formule V et leurs mélanges : 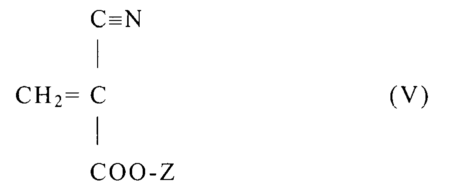 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

Par milieu cosmétiquement acceptable, on entend un milieu compatible avec les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique et l'alcool béhénylique, les acides gras en C₁₀-C₃₀ tels que l'acide laurique et l'acide stéarique, les amides gras en C₁₀-C₃₀ tels que le diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 10⁵ Pa (760mm de Hg).

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, tel que, par exemple, des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, et des épaississants minéraux ou organiques tels que le benzylidène-sorbitol et les N-acylaminoacides.

Ces agents peuvent être éventuellement encapsulés. La capsule peut être de type polycyanoacrylate.

De préférence, le dispositif aérosol à deux compartiments est constitué par un bidon aérosol externe comportant une poche interne soudée hermétiquement à une valve. La composition est introduite dans la poche interne et un gaz comprimé est introduit entre la poche et le bidon à une pression suffisante pour faire sortir le produit sous forme d'un spray à travers l'orifice d'une buse. Un tel dispositif est commercialisé sous le nom EP SPRAY par la société EP-SPRAY SYSTEM SA.

La composition contenue dans le dispositif aérosol selon l'invention peut être utilisée pour le traitement des cheveux, et plus particulièrement pour leur coiffage. La composition est pulvérisée sur les cheveux en présence d'un agent nucléophile.

Le procédé de traitement cosmétique selon l'invention comprend la pulvérisation d'une composition telle que définie ci-dessus, contenue dans le dispositif aérosol de l'invention, sur les cheveux, en présence d'un agent nucléophile tel que défini ci-dessous.

Les agents nucléophiles susceptibles d'initier la polymérisation anionique sont des systèmes connus en eux-mêmes, capables de générer un carbanion au contact d'un monomère électrophile. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O Ph représentant le groupe phényle ; Ar représentant un groupe aryle et R représentant un groupe alkyle en C₁-C₁₀.

Les agents nucléophiles particulièrement préférés sont les ions hydroxyle, notamment ceux présents dans l'eau. Cette eau peut être apportée par une humidification préalable des cheveux.

Selon un mode préféré de réalisation, la composition comprenant le monomère électrophile est exempte d'agent nucléophile.

Selon un autre mode préféré de réalisation, l'agent nucléophile est apporté par une seconde composition, appliquée sur ou sous le dépôt formé par l'application de la composition comprenant le monomère électrophile.

Il est également possible, afin de moduler la cinétique de réaction, de préalablement humidifier les cheveux, à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les cheveux, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des cheveux, par transformation chimique de la matière kératinique de la fibre capillaire.

A titre d'exemple de transformation chimique, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6 224 622.

Afin d'améliorer entre autres l'adhésion du poly(cyanoacrylate) formé in situ, on peut pré-traiter la fibre capillaire avec tous types de polymères, ou réaliser un traitement capillaire avant application de la composition de l'invention, comme une coloration directe ou d'oxydation, une permanente ou encore un défrisage.

L'application des compositions contenues dans le dispositif aérosol conforme à l'invention peut être suivie ou non d'un rinçage.

L'exemple suivant est donné à titre illustratif de la présente invention.

Dans l'exemple suivant, toutes les quantités sont indiquées en pour cent en poids par rapport au poids total de la composition, sauf indication contraire.

### Exemple 1 :

On a préparé la composition suivante :

| | |
|---|---|
| 2-cyanoacrylate de n-octyle ⁽¹⁾ | 10 g |
| Cyclopentadiméthylsiloxane ⁽²⁾ | 90 g |
| ⁽¹⁾ : RITE LOK CON895, commercialisé par la Société CHEMENCE | |
| ⁽²⁾ : Dow Corning 245 Fluid | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

### Exemple 2 :

On a préparé la composition suivante :

| | |
|---|---|
| Méthylheptylcyanoacrylate ⁽¹⁾ | 10 g |
| Cyclopentadiméthylsiloxane ⁽²⁾ | 90 g |

| | |
|---|---|
| ⁽¹⁾ commercialisé par la Société CHEMENCE | |
| ⁽²⁾ : Dow Corning 245 Fluid | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

### Exemple 3 :

On a préparé la composition suivante :

| | |
|---|---|
| Méthylheptylcyanoacrylate ⁽¹⁾ | 10 g |
| Cyclopentadiméthylsiloxane ⁽²⁾ | 89.75 g |
| Acide acétique | 0.25g |

| | |
|---|---|
| ⁽¹⁾ commercialisé par la Société CHEMENCE | |
| ⁽²⁾ : Dow Corning 245 Fluid | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

### Exemple 4 :

On a préparé la composition suivante :

| | |
|---|---|
| Ethoxyethylcyanoacrylate ⁽¹⁾ | 10 g |
| Cyclopentadiméthylsiloxane ⁽²⁾ | 89 g |
| Acide acétique | 1 g |

| | |
|---|---|
| ⁽¹⁾ EO 460 commercialisé par la Société Tong Shen | |
| ⁽²⁾ : Dow Corning 245 Fluid | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

### Exemple 5 :

On a préparé la composition suivante :

| | |
|---|---|
| Butylcyanoacrylate ⁽¹⁾ | 10 g |
| Cyclopentadiméthylsiloxane ⁽²⁾ | 89 g |
| Acide acétique | 1g |

| | |
|---|---|
| ⁽¹⁾ B 60commercialisé par la Société Tong Shen | |
| ⁽²⁾ : Dow Corning 245 Fluid | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

### Exemple 6 :

On a préparé la composition suivante :

| | |
|---|---|
| Ethylhexylcyanoacrylate ⁽¹⁾ | 10 g |
| Cyclopentadiméthylsiloxane ⁽²⁾ | 90 g |

| | |
|---|---|
| ⁽¹⁾ O-60 commercialisé par la Société Tong Shen | |
| ⁽²⁾ : Dow Corning 245 Fluid | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

### Exemple 7 :

On a préparé la composition suivante :

| | |
|---|---|
| Méthylheptylcyanoacrylate ⁽¹⁾ | 9 g |
| Ethylhexylcyanoacrylate ⁽³⁾ | 1 g |
| Cyclopentadiméthylsiloxane ⁽²⁾ | 90 g |

| | |
|---|---|
| ⁽¹⁾ commercialisé par la Société CHEMENCE | |
| ⁽²⁾ : Dow Corning 245 Fluid | |
| ⁽³⁾ : O-60 commercialisé par la société Tong Shen | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

### Exemple 8 :

On a préparé la composition suivante :

| | |
|---|---|
| Méthylheptylcyanoacrylate ⁽¹⁾ | 7 g |
| Butylcyanoacrylate ⁽³⁾ | 3 g |
| Cyclopentadiméthylsiloxane ⁽²⁾ | 90 g |

| | |
|---|---|
| ⁽¹⁾ commercialisé par la Société CHEMENCE | |
| ⁽²⁾ : Dow Corning 245 Fluid | |
| ⁽³⁾ : B-60 commercialisé par la société Tong Shen | |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux mouillés. La pulvérisation se fait sous forme d'un spray doux.

On obtient, après séchage, une chevelure présentant un bon maintien.

## Revendications

1. Dispositif aérosol à deux compartiments comprenant :
(1) dans un premier compartiment, une composition cosmétique comprenant dans un milieu cosmétiquement acceptable, au moins un monomère électrophile, et
(2) dans un deuxième compartiment, un gaz comprimé,
lesdits premier et deuxième compartiments assurant, lors de l'emploi du dispositif, une séparation mécanique permanente à l'intérieur de celui-ci entre la composition cosmétique et le gaz comprimé.

2. Dispositif aérosol selon la revendication 1, **caractérisé en ce que** le gaz comprimé est choisi parmi l'air, l'azote, le gaz carbonique et leurs mélanges.

3. Dispositif aérosol selon la revendication 2, **caractérisé en ce que** le gaz comprimé est l'air.

4. Dispositif aérosol selon la revendication 1, 2 ou 3, **caractérisé en ce que** la pression du gaz comprimé est comprise entre 0,1 et 1,2 MPa.

5. Dispositif aérosol selon la revendication 4, **caractérisé en ce que** la pression du gaz comprimé est comprise entre 0,9 et 1,1 MPa.

6. Dispositif aérosol selon l'une quelconque des revendications précédentes, **caractérisé en ce que** le ou les monomères électrophiles répondent à la formule : 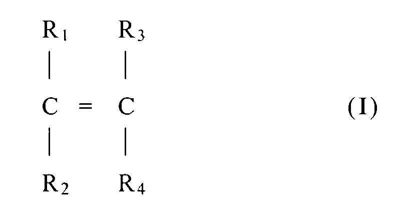 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR, -COOR, -COR, - SH, -SR, -OH et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, -S(R)₂⁺, - SH₂⁺, -NH₃⁺, -NO₂, -SO₂R -C≡N, -COOH, -COOR, -COSR, -CONH₂, - CONHR,-F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle et aryloxy.
R désigne un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR', -COOR', -COR', -SH, -SR', -OH, les atomes d'halogène, et un résidu de polymère pouvant être obtenu par polymérisation radicalaire ou par polycondensation ou par ouverture de cycle, R' désignant un groupe alkyle en C₁-C₁₀.

7. Dispositif aérosol selon la revendication 6, **caractérisé en ce que** le ou les monomères électrophiles sont choisis parmi les composés de formule : 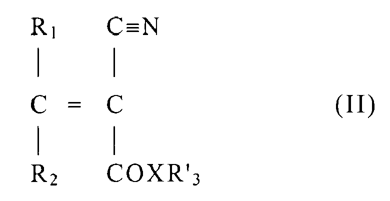 X désignant NH,S,O,
R₁ et R₂ sont tels que définis dans la revendication 6,
R'₃ pouvant désigner un atome d'hydrogène ou un groupe R tel que défini dans la revendication 6.

8. Dispositif aérosol selon la revendication 7, **caractérisé par le fait que** le ou les monomères sont choisis parmi les 2-cyanoacrylates de polyfluoroalkyle en C₁₋₂₀, les cyanoacrylates d'alkyle en (C₁-C₁₀) ou d'(alcoxy en C₁-C₄)(alkyle en C₁-C₁₀).

9. Dispositif aérosol selon la revendication 8, **caractérisé en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

10. Dispositif aérosol selon la revendication 9, **caractérisé en ce que** le ou les monomères électrophiles répondent à la formule (V) : 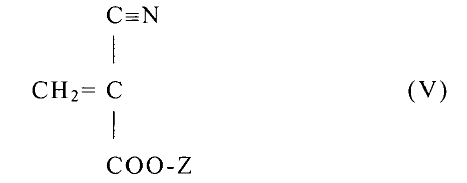 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

11. Dispositif aérosol selon l'une quelconque des revendications précédentes, **caractérisé en ce que** le ou les monomères électrophiles sont fixés de façon covalente sur des supports tels que des polymères, des oligomères ou des dendrimères.

12. Dispositif aérosol selon l'une quelconque des revendications précédentes, **caractérisé en ce que** la quantité de monomère électrophile va de 0,001 à 80 % en poids, de préférence de 0,1 à 40 % en poids par rapport au poids total de la composition cosmétique.

13. Dispositif aérosol selon l'une quelconque des revendications précédentes, **caractérisé en ce que** le milieu cosmétiquement acceptable est anhydre.

14. Dispositif aérosol selon la revendication 13, **caractérisé en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

15. Dispositif aérosol selon l'une quelconque des revendications précédentes, **caractérisé en ce que** la composition contient des inhibiteurs de polymérisation.

16. Dispositif aérosol selon la revendication 15, **caractérisé en ce que** les inhibiteurs sont des inhibiteurs de polymérisation anioniques et/ou radicalaires.

17. Dispositif aérosol selon la revendication 15 ou 16, **caractérisé en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges.

18. Dispositif aérosol selon l'une quelconque des revendications 15 à 17, **caractérisé en ce que** les inhibiteurs de polymérisation sont présents en des quantités allant de 10 ppm à 20 % par rapport au poids total de la composition.

19. Dispositif aérosol selon l'une quelconque des revendications précédentes, **caractérisé en ce que** la composition contient en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensio-actifs anioniques, cationiques, non ioniques ou amphotères, les polymères fixants ou non, les polyols, les protéines, des vitamines, les colorants directs ou d'oxydation, les agents nacrants et des épaississants minéraux ou organiques.

20. Dispositif aérosol selon la revendication 19, **caractérisé en ce que** l'agent est encapsulé.

21. Utilisation de la composition contenue dans le dispositif aérosol selon l'une quelconque des revendications précédentes, pour le traitement cosmétique des cheveux.

22. Utilisation selon la revendication 21, en présence d'un agent nucléophile.

23. Utilisation selon la revendication 22, **caractérisée en ce que** l'agent nucléophile est choisi parmi les composés moléculaires, les oligomères, les dendrimères ou polymères possédant des fonctions nucléophiles choisies parmi : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

24. Utilisation selon la revendication 23, **caractérisée en ce que** l'agent nucléophile comprend des ions hydroxyle, notamment ceux présents dans l'eau.

25. Utilisation selon l'une quelconque des revendications 21 à 24, pour le coiffage des cheveux.

26. Procédé de traitement cosmétique des cheveux, comprenant la pulvérisation sur les cheveux d'une composition contenue dans le dispositif aérosol selon l'une quelconque des revendications 1 à 20, en présence d'un agent nucléophile.

27. Procédé selon la revendication 26, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃-, ClO₄⁻ et H₂O, Ph représentant le groupe phényle, Ar représentant un groupe aryle et R représentant un groupe alkyle en C₁-C₁₀.

28. Procédé selon la revendication 27, **caractérisé en ce que** l'agent nucléophile comprend des ions hydroxyle, notamment ceux présents dans l'eau..

29. Procédé selon l'une quelconque des revendications 26 à 28, **caractérisé en ce que** l'on applique la composition sur les cheveux préalablement humidifiées à l'aide d'une solution aqueuse dont le pH a été ajusté à l'aide d'une base, d'un acide ou d'un mélange acide/base.

30. Procédé selon l'une quelconque des revendications 26 à 28, **caractérisé en ce que** les cheveux sont pré-imprégnés à l'aide d'un agent nucléophile différent de l'eau.

31. Procédé selon l'une quelconque des revendications 26 à 28, **caractérisé en ce que** les cheveux sont préalablement réduits avant application de la composition.

32. Procédé selon l'une des revendications 26 à 31, **caractérisé en ce que** l'application de la composition est suivie d'un rinçage.
